# EUROPEAN PATENT APPLICATION

(11) **EP 1 774 899 A1**
(43) Date of publication of application: **18.04.2007**
(21) Application number: 05774295.9
(22) Date of filing: 09.06.2005
(51) Int. Cl.: A61B 3/107, A61B 18/20, A61F 9/01

(54) **METHOD OF PREVENTING THE INDUCTION OF ABERRATIONS IN LASER REFRACTIVE SURGERY SYSTEMS**

(30) Priority: 11.06.2004 ES 200401441
(71) Applicant: CONSEJO SUPERIOR DE INVESTIGACIONES CIENT FICAS, 28006 Madrid (ES)
(72) Inventor: MARCOS CELESTINO, Susana INSTO. DE ÓPTICA, C/ Serrano, 121 E-28006 MADRID (ES); DORRONSORO DÍAZ, Carlos INSTO. DE ÓPTICA, C/ Serrano, 121 E-28006 MADRID (ES); CANO REOL, Daniel INSTO. DE ÓPTICA, C/ Serrano, 121 E-28006 MADRID (ES)
(74) Representative: Ungria Lopez, Javier
(86) International application number: PCT/ES2005/070087
(87) International publication number: WO 2005/122873

(57) **Abstract**

The invention relates to a method of preventing the induction of aberrations in laser refractive surgery systems. Standard laser refractive surgery systems successfully correct low-order refractive errors (myopia, hypermetropia and astigmatism), but induce spherical aberration and, by extension, other high-order aberrations, which result in a worsening of vision quality. Said increase in spherical aberration is due to the shape of the cornea, and not inherent in the theoretical ablation profile, and, as a result, the problem is, in principle, common to all laser systems. The invention relates to a systematic method which can be used with any system and ablation profile in order to obtain a profile correction factor. The correction factor, which is specific to each system, can be applied in order to prevent the induction of spherical aberration and, in this way, improve the optical and visual quality of patients following surgery compared to surgery performed with standard systems. Moreover, the inventive method can be used to improve the production of lenses with controlled high-order aberrations using laser systems.

## Description

### Field of the invention

This invention refers in general to the field of ophthalmology, and in particular to improving the methods for modifying the shape of the cornea (and by extension of any lens) by means of laser ablation systems for the correction of refractive errors and other optical defects.

### Prior art of the invention

The main optical components of the human eye are the cornea and the crystalline lens, whose function is to create the images of the objects of the outside world on the retina. More than 60% of the population suffer from ocular refractive errors, which reduce the quality of image on the retina. Conventional refractive errors are myopia, hypermetropia and astigmatism, also known as low-order optical aberrations. Myopia and hypermetropia are caused by the length of the eye not being properly adjusted to the power of the ocular optics, so that the images projected on the retina are out of focus. Astigmatism is caused by the meridional variation of the ocular optical power and it produces an asymmetric blurring of the images on the retina. Apart from conventional refractive errors (myopia, hypermetropia and astigmatism), all eyes have other optical defects known as high-order optical aberrations. One of the most important high-order optical aberrations is spherical aberration (change of power with pupil diameter) which gives rise to blurring of the image and halo vision.

Refractive errors are traditionally compensated both with ophthalmic lenses and with contact lenses. As an alternative to these correction methods, corrective surgical procedures of the incisional type, such as radial keratotomy, appeared in the '80s, They have recently been replaced by photorefractive keratectomy (PRK) and laser assisted *in-situ* keratomileusis (LASIK) which modify the shape of the cornea in order to thereby change its power and compensate the refractive errors. These last two procedures use an excimer laser for forming the cornea in order to remove tissue by means of ablation. While in PRK surgery the ablation commences on the surface layers of the cornea (first the epithelium and then the Bowman layer), in LASIK surgery those layers are not ablated since a microkeratome creates a surface lamina of corneal tissue which is removed prior to the ablation and replaced afterwards so that just the stroma is ablated.

In refractive procedures known as standard, the ablation pattern is based on the Munnerlyn function (Munnerlyn C, Koons S, Marshall J. Photorefractive keratectomy: a technique for laser refractive surgery. J Cataract Refract Surg 1988; 14:46-52), whose values are the ablation depths at each point of the cornea. The Munnerlyn function is the subtraction of two spherical surfaces representing the corneal surfaces before and after the ablation. The difference in powers of both spheres is the power it is wished to correct. The Munnerlyn ablation pattern can be used for correction of myopia (reducing the power of the cornea) and for the correction of hypermetropia (increasing the power of the cornea). This ablation pattern can also be used for correction of astigmatism by introducing a meridional dependence on power into the Munnerlyn function. A parabola is frequently used as an approximation to the Munnerlyn function (Jiménez J, Anera R, Jiménez del Barco L. Equation for corneal asphericity after corneal refractive surgery. J Refract Surg. 2003:65-69; Lin J. Critical review on refractive surgical lasers. Optical Engineering 1995;34:668-675). Said parabolic formula is obtained by truncating the Taylor development of the Munnerlyn function. As well as the Munnerlyn and parabolic ablation patterns, other ablation patterns have been proposed that are defined with biconic surfaces (Schwiegerling J. Snyder R. Custom photorefractive keratectomy ablations for the correction of spherical and cylindrical refractive error and higher-order aberration. Journal of the Optical Society of America A 1998; 15:2572-2579) or with individual optical aberrations (personalised ablation patterns: Manns F, Ho A, Parel J, Culbertson W. Ablation profiles for wavefront-guided correction of myopia and primary spherical aberration. J Cataract Refract Surg 2002;28:766-774). Moreover, ablations with multifocal algorithms have been proposed and carried out (Odrich N, Greenberg K, Legerton J, Munnerlyn C, Schimmick J. Method and systems for laser treatment of presbyopia using offset imaging. United States Patent # 6,663,619; VISX Incorporated, 2003). The ablation pattern designed with biconic surfaces, as well as the apical radii of curvature, considers corneal asphericities in such a way that permits control not just of the change of power but also of the corneal asphericity after the ablation (and therefore the spherical aberration). Corneal asphericity is defined as the asphericity Q of the conic surface x² + y² + (1 + Q) z² - 2zR = 0 which provides the best fit for the corneal surface, where R is the apical radius of curvature and (x,y,z) arc Cartesian coordinates. The average asphericity of pre-operative corneas is slightly negative (Q = -0.26), indicating greater curvature in the centre of the cornea than in the periphery. This asphericity provides a slightly positive corneal spherical aberration, which tends to be compensated in young subjects with the negative spherical aberration of the lens. A cornea that is free of spherical aberration would have an asphericity of -0.52 (Atchison DA, Smith G. Optics of the Human Eye. Oxford: Butterworth-Heinemann, 2000). The personalised ablation pattern exploits the possibility offered by flying spot excimer laser systems of eliminating tissue asymmetrically. Based on the prior measurement of the patient's map of ocular aberrations, this in theory permits a pattern to be formed on the cornea such that post-operative ocular aberrations approximate to zero. Algorithms of this type have been proposed for the manufacture by means of flying spot excimer laser systems of customized phase plates or contact lenses for the correction of the patient's ocular aberrations (Chernyak D, Campbell C. Systems for the design, manufacture, and testing of custom lenses with known amounts of high-order aberrations. JOSA A 2003:20; 2016-2021). For this, account has to be taken of the differences in refractive index of the cornea and of the plastic material used. Algorithms are currently starting to be applied which produce a multifocal ablation pattern for use in patients with presbyopia (loss of focusing capacity which affects the entire population starting from the age of 45).

Clinical experience shows that PRK and LASIK surgery in general satisfactorily eliminate the conventional refractive errors of patients. Nevertheless, it has been experimentally demonstrated that spherical aberration is significantly increased (a factor of close to 4 on average in a group of patients with 22 D) with standard LASIK refractive surgery for myopia (Moreno-Barriuso E, Merayo-Lloves J, Marcos S., et al. Ocular aberrations before and after myopic corneal refractive surgery: LASIK-induced changes measured with Laser Ray Tracing. Invest. Oph. Vis. Sci. 2001:42;1396-1403). This increase is mainly produced in the cornea ( Marcos S, Barbero B, Llorente L, Merayo-Lloves J. Optical response to LASIK for myopia from total and corneal aberration measurements. Invest. Oph. Vis. Sci. 2001:42;3349-3356) and produces a diminution in the visual function in terms of sensitivity to contrast (Marcos S. Aberrations and visual Performance following standard laser vision correction. J. Refract. Surgery 2001:17:596-601), which is manifested in the form of nocturnal halos and other visual artifacts which can sometimes be very annoying for the patient.

The increase in corneal spherical aberration is associated with an increase in corneal asphericity. Some analytical and theoretical studies have looked at the possible causes of the increase in spherical aberration inherent to the standard ablation pattern (Jiménez J, Anera R, Jiménez del Barco L. Equation for corneal asphericity after corneal refractive surgery. J Refract Surg. 2003:65-69; Gatinel D. Hoang-Xuan T, Azar D. Determination of corneal asphericity after myopia surgery with the excimer laser: a mathematical model. Invest. Ophthalmol. Vis. Sci. 2001;42:1736-1742). Post-operative corneal topographies, obtained by means of computational subtraction of the theoretical Munnerlyn ablation pattern from the pre-operative corneal topography in real patients do not show an increase in the corneal asphericity. The same type of computational studies show an increase in the corneal asphericity obtained by means of the subtraction of the parabolic ablation pattern, though much less than the increase of the corneal asphericity in real data. The cause of the experimentally observed increase in corneal asphericity in patients is therefore not found in the theoretical definition of the algorithms.

Various authors have suggested that the increase in corneal asphericity could be caused by variations in the efficiency of the ablation due to changes in the angle of incidence of the laser on the cornea (Mrochen M, Seiler T. Influence of corneal curvature on calculation of ablations patterns used in photorefractive laser surgery. J Refract Surg. 2001;17:S584-S587; Jiménez J, Anera R, Jiménez del Barco L, Hita E. Effect on laser-ablation algorithms of reflection losses and nonnormal incidence on the anterior cornea. Appl. Phys. Lett. 2002:81(8):1521-1523). The angle of incidence of the laser increases from the apex to the periphery of the cornea, and, consequently, the energy per unit of corneal surface decreases due to the fact that both the reflected energy (Fresnel laws) and the illuminated area increase. Exclusively theoretical calculations have provided a generic factor K which depends on the angle of incidence, and therefore on the corneal position. This factor represents the efficiency of the ablation and its values lie between 0 and 1, being 1 when the efficiency is total (the expected amount of tissue is ablated). For example, if, at a point of the cornea, the K factor is 0.9, the efficiency of the ablation at that point is 90%, and therefore only 90% of the expected tissue will be ablated. The incorporation of this theoretical factor into computational simulations of the post-operative corneal topography introduces an increase in the corneal asphericity, but it does not explain the clinical values, which on average are a factor of x 1.47 higher (Cano D, Barbero B, Marcos S. Computer application of laser ablation patterns on real corneas and comparison with surgical outcomes. Journal of the Optical Society of America A. (in press) 2004).

Alternatively, it has been suggested that the increase in corneal asphericity with LASIK refractive surgery is due to biomechanical corneal factors, Patent application US 2003/0208190 A1 makes reference to the incorporation of biomechanical corneal data in refractive treatments.

The present invention is based on measurements made on artificial corneas of plastic material ablated with refractive surgery lasers, as will be described below. These ablated plastic corneas also show the effects of asphericity described in real corneas, and it can therefore be deduced that the biomechanical or biological effects have a magnitude less than what has been attributed to them so far.

The present invention refers to a method for assessing the contribution of changes of efficiency of the laser on a plastic spherical surface (analogous in shape to the cornea) so that it can be incorporated into any laser ablation pattern for treatment of the cornea. Unlike the case of a generic theoretical factor, this method will permit any laser to be calibrated, with its particular features concerning type, shape, distribution, uniformity and overlapping of pulses. The method will furthermore be applicable to refractive surgery by means of standard patterns (based on the Munnerlyn theoretical equation or approximations) for the correction of myopia, hypermetropia and astigmatism, as well as for more sophisticated theoretical ablation patterns, including personalised ablation patterns (customised to the aberrations of the patient) or multifocal ablation patterns (for the treatment of presbyopia). The application of this factor will prevent the induction of virtually all spherical aberration. Likewise, a similar protocol, with differences in the scale factors associated with the differences in the rate of ablation between different types of material or the cornea, will be able to be applied in the manufacture by means of laser ablation of contact lenses or lenses in general for the correction or induction of refractive errors and other high-order optical aberrations, both in eyes and in instruments. The method that is proposed can be used in the metrology of aberrations, such as for example in the manufacture of aberration standards or calibrations for aberrometers or topographs, or in the precise transfer of primary patterns to secondary patterns.

The present invention proposes the production of cornea models (or generic lenses) made of plastic, their laser ablation by means of standard PTK, LASIK or PRK profiles, the obtaining of the ablation profile by means of subtraction of the elevation topography following the ablation from the topography prior to the ablation (prior polishing of the surface and correction of alignment errors), the obtaining of scale factors of the rate of ablation in the plastic material compared to the rate of ablation in corneal tissue (or in another different material), and the obtaining of the correction factor as a quotient of the theoretical ablation profile (or measured on flat surfaces) and the ablation profile measured in the cornea model (or generic lens). The optimum ablation pattern will be obtained as a product of the theoretical ablation pattern, the correction factor for the geometry and the scale factor for the rate of ablation.

### General description of the invention

The present invention implies an improvement in the technique of corneal refractive surgery, in that the application of the inventive method prevents the induction of spherical aberration (and by extension, other aberrations) observed in patients operated on with LASIK or PRK refractive surgery. Preventing the increase in said spherical aberration will imply an improvement in optical quality following surgery, and therefore of the visual quality in those patients compared to that obtained by means of conventional methods. The same method can be extended to optimisation of the ablation patterns for the customised forming of lenses by means of excimer laser.

Consequently, the objective of the present invention is to provide a method for obtaining a correction factor for the changes in efficiency of the laser used for forming the cornea (or other lenses), due to the geometry of the cornea (or lens). Generic theoretical factors proposed in the literature do not manage to explain the clinical data obtained in patients. Also, the contribution of biomechanical effects in the cornea has been overvalued in previous works. The correction factor will be specific for each laser unit, in such a way that the inventive method will permit each manufacturer to obtain the factor corresponding to each of his systems. This factor will take account of the possible differences between units such as laser power, alignments, vignetting, shape or uniformity of the spot. Different correction factors will be able to be obtained for different work modes of the laser if necessary, or the method could be adapted so that it can be used periodically by the user or operator of the system. The correction factors obtained in this way will be applicable to any theoretical ablation pattern for forming of the cornea (or generic lens).

Another objective of the present invention is to provide a physical model of the cornea in a plastic material and the methods for characterising the surfaces before and after the laser ablation.

A further objective of the present invention is to provide a method for the reliable obtaining of the ablation profile starting from topographical data before and after the ablation, eliminating artifacts relating to errors of positioning of the surface in measuring the corneal topography, systematic errors of the corneal topography in the periphery of the cornea or errors of fit of the reference surface.

Another objective of the present invention is to extrapolate the information obtained on the plastic material to the cornea (or to a lens of another material).

A further objective of the present invention is the definition of the method for optimisation of the ablation patterns for laser treatment of the cornea (or other lens), based on the correction factor and scale factor obtained for the physical model of the cornea.

### Detailed description of the invention

The present invention is introduced as a calibration protocol of lasers for the treatment of the cornea (or any lens in general) and fit of the ablation algorithms, consisting of the following stages:
1. Manufacture of a physical model in plastic (for example in PMMA) with the shape of the cornea. The model must be constructed in a material with a response to the ablation by excimer laser of an order of magnitude similar to that of the corneal tissue, and be polished such that it has a reflecting surface. The shape of the surface will be assessed by means of a corneal topograph.
2. Ablation of the surfaces described in stage 1 by means of PTK (phototherapeutic keratectomy) for a standard ablation depth and treatment zone. In theory, this method eliminates a constant thickness of tissue by the application of the same energy at each corneal position, though changes in the efficiency of the laser with the corneal position will produce a nonuniform ablation of the tissue. The shape of the resulting surface will be assessed by means of a corneal topograph. The surface will be polished slightly in order to eliminate possible irregularities caused by the ablation process, but without affecting the overall shape of the surface. The shape of the surface will be assessed by means of a corneal topograph.
3. As an alternative to stage 2, ablation of the surfaces described in stage 1 by means of conventional LASIK or PRK for different corrections of ametropias, such as myopia. The procedure will be carried out for different treatment zones. In theory, this procedure forms the surface defined by the theoretical ablation pattern (for example, the Munnerlyn pattern or parabolic Munnerlyn pattern), though the changes in the laser efficiency with the corneal position will produce a discrepancy with respect to the theoretical profile. The shape of the resulting surface will be assessed by means of a corneal topograph. The surface will be polished slightly in order to eliminate possible irregularities caused by the ablation process, but without affecting the overall shape of the surface. The shape of the surface will be assessed by means of a corneal topograph.
4. Assessment of the real ablation profile transmitted to the cornea as the subtraction of the profile following the ablation less the profile prior to the ablation. Account will be taken of the subtraction algorithms for decentrations and misalignments between the PRE and POST ablation topography, which will be even more important than in real eyes owing to the fact that artificial corneas are not going to have the references existing in eyes: line of sight and natural pupil. The subtraction will be made along the axis in which the laser ablation was applied and not along the z axis of the topography. The vertical positioning (piston) between both topographies will be adjusted on the basis of the non-ablated zone of the POST topography compared to the same zone of the PRE topography. For this, a prior assessment will be made of the errors in the periphery of the cornea of the topograph used. The resulting ablation profile will be known as P_{PTK} or R_{REF} depending on whether the treatment carried out is PTK or refractive (LASIK or PRK).
5. Obtaining of the conversion scale factor between nominal ablation depths in the cornea (provided by the manufacturer of the excimer laser for treatment of the cornea) and ablation depths in the manufacturing material for the physical model. Said scale factor, E, will be the slope of linear for regression of the data on the nominal ablation depth in the cornea compared to the data on the maximum ablation depth measured at the apex (for the physical model), for various corrections. This scale factor E will be calculated for each optical zone. This calibration can be extrapolated for other materials different from the manufacturing material of the physical model of the cornea, obtaining a scale factor E'. The conversion factor for ablation depth data with respect to the physical model proposed, for forming of lenses in other materials (instead of the cornea), will be E/E'.
6. Obtaining of the geometric correction factor K, which represents the efficiency of the ablation at each point of the cornea. This factor K is calculated as P_{SPHERE} /P_{THEORY} where P_{SPHERE} is the ablation pattern measured on a spherical surface and P_{THEORY} is the theoretical ablation factor. In the case of PTK, the theoretical pattern is generally constant. Given that both P_{THEORY} and P_{SPHERE} are functions of the corneal position, K is also a function of the corneal position. It is understood that the theoretical ablation position is known by the manufacturer. If that pattern is not known, then it can be obtained by laser ablation (with the same pattern) of a flat surface of the same manufacturing material used for the physical model of the cornea, with that profile being assessed by means of contact profilometry.
7. Optimisation of any theoretical ablation pattern P_{THEORY} (for example, Munnerlyn, biconic or customised to the patent's pre-operative aberrations) by means of applying the scale and correction factors described here: P_{FINAL} = P_{THEORY} * K * E. For forming of lenses in another material different from the cornea, E will be replaced by E/E' . Said pattern P_{FINAL} will be free of the increase in spherical aberration associated with the application of the theoretical pattern P_{THEORY} without considering the correction factor.

### Detailed description of the figures

Figure 1. 3-D ablation profile in a model made from PMMA following nominal ablation of -6 and -12 dioptres; x indicates the horizontal position in the cornea, y the vertical position in the cornea and z the ablation depth in the cornea.
Figure 2. Ablation depth measured in PMMA (X) compared to the nominal value of the ablation depth in the cornea. The linear fit of the data (coefficient of r = 0.995) provides the scale factor between the model and the cornea: 2.49.
Figure 3: Post-operative corneal asphericity Q as a function of the correction of myopia in dioptres for real patients (diamonds), physical model made of PMMA (circles) and simulation following application of the correction factor (solid line) in combination with the standard ablation profile programmed in the laser.
Figure 4. High-order aberration patterns in a patient with a pre-operative myopia of -5 dioptres: A) Before LASIK surgery. B) Simulation following LASIK surgery with ablation pattern personalised for correction of pre-operative aberrations without inclusion of the correction factor K. C) Simulation as in B, but with application of the correction factor obtained according to the described method.

### Example of the embodiment of the invention

As a practical case of embodiment of the invention, which it must be understood has no limiting nature on it, described below is a possible method of application, carried out for:
1) A physical PMMA model of the cornea of radius 8 mm, made of extruded PMMA and polished using a lathe and precision optical tools.
2) Laser ablation for correction of myopia using the flying spot laser, Technolas 217 Z from Bausch & Lomb.
3) Computerised corneal topograph from Humphrey-zeiss (Atlas model).
4) Analysis of data with a computer by means of the Matlab mathematical program.

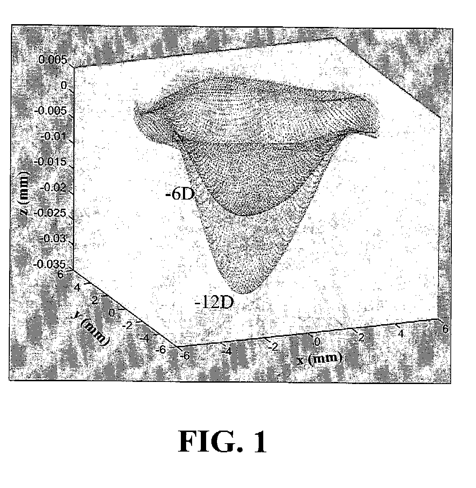

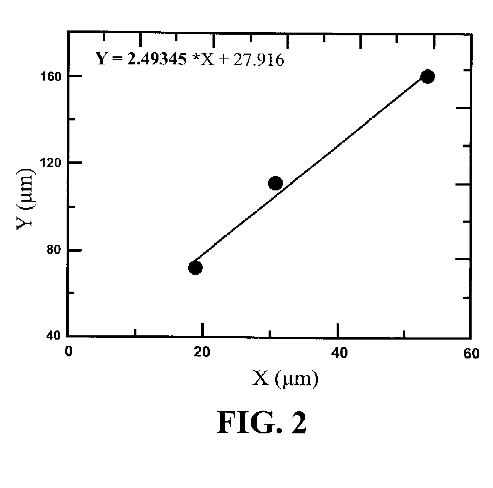

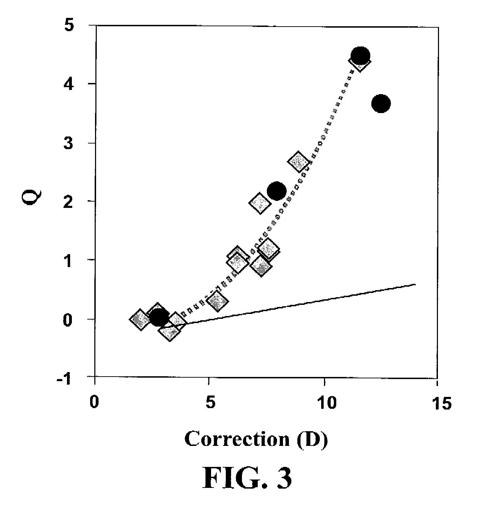

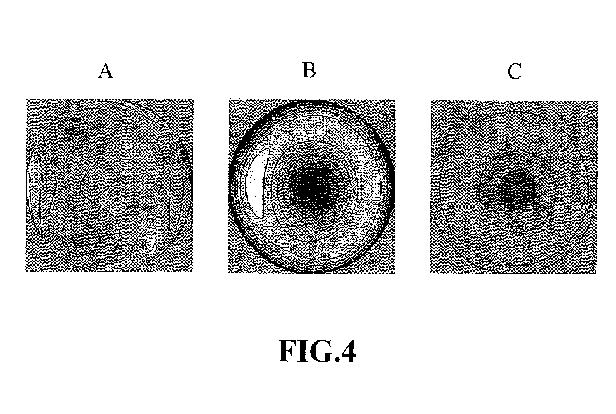

In this case, three cornea models produced with PMMA were ablated, with nominal corrections of -3, -6 and -12 dioptres and optical zone 6 mm.
The topographies of the surfaces were measured before and after the ablation and the ablation profiles were calculated as the difference in the elevation of the surface before and after treatment. Figure 1 shows the ablation profiles obtained for a correction of -6 and -12 dioptres respectively, following application of the method described in stage 4.
The scale factor between the ablation depth in the cornea and the ablation depth in PMMA was obtained following the method described in 5. Figure 2 shows the regression line between ablation depths measured in the cornea models and the ablation depth in the cornea. A scale factor was obtained of E = 2.5.
The correction factor K was obtained as the inverse of the ablation profile shown in figure 1, multiplied by the nominal profile programmed in the laser for that same correction. The nominal patterns are not public knowledge. The pattern programmed in the laser was obtained by means of ablation of a flat surface of PMMA, for the same correction and optical zone. This profile was closer to the parabolic approximation of the Munnerlyn algorithm than to the exact Munnerlyn pattern. This method was repeated for two different corrections, with similar K factors being found.
The corneal asphericity resulting from applying the algorithm programme in the laser was compared to the corneal asphericity resulting from applying the same algorithm, optimised with the K correction factor obtained by means of the physical model of the cornea forming the object of the invention. Figure 3 shows the post-operative corneal asphericity in real patients, with the asphericity (after applying the scale factor E) of the physical model in PMMA following application of the laser ablation, with the simulation of the corneal asphericity after having introduced the correction factor forming the object of this invention in the ablation method, as a function of the corrected refractive error.
Finally, the results have been simulated of the application of a theoretical ablation profile designed for the correction of real ocular aberrations of a patient (in Figure 4A, in which the defocusing term has been eliminated in order to view the high-order aberrations), without the application of the correction factor forming the object of this invention and following the application of the correction factor obtained in this example, according to the method described in stage 7. The post-operative aberrations pattern shows an increase in spherical aberration when that factor is not taken into account, which masks any correction of the pre-operative aberrations (Figure 4B), while the introduction of the correction factor shows a reduced aberrations pattern (Figure 4C).

## Claims

1. Method for determining a geometric correction factor K to the theoretical ablation profiles for the treatment of refractive errors, low and high order, of the cornea or the manufacture of lenses of another material by means of laser which prevents the induction of spherical aberration of the cornea, or lens, and obtaining of an optimised ablation profile, **characterised by** the
i. Generation of a model of artificial cornea (or generic lens) on which the laser ablation is applied
ii. Topographical measurement and specific analysis of the data of the surface before and after the ablation
iii. Obtaining the factor by which the ablation profile has to be multiplied for each coordinate in the cornea or lens to manufacture in order to obtain the optimum treatment, correcting the discrepancies with respect to the theoretical profile owing to the geometry of the cornea, or lens to treat.

2. Method for the generation of a model of artificial cornea according to claim 1, section i, **characterised by** the manufacture of a spherical surface of plastic material of radius similar to that of the cornea, or other lens of comparable radius, and by the ablation of said surface according to the method known as PTK, phototherapeutic keratectomy, which seeks to eliminate constant thicknesses of tissue for different ablation depths.

3. Method for the generation of a model of artificial cornea according to claim 1, section i, **characterised by** the manufacture of a spherical surface of plastic material of radius similar to that of the cornea, or other lens of comparable radius, and by the ablation of said surface according to the conventional LASIK or PRK method, for different corrections of ametropia and different optical zones.

4. Method for obtaining the ablation profile on spherical surfaces according to claim 1, section ii, for the surfaces **characterised in** claims 2 and/or 3, **characterised by** the
i. Measurement, by means of a corneal topography system, of the elevation of the surface in each point prior to ablation,
ii. Measurement, by means of a corneal topography system, of the elevation of the surface following ablation after application of a slight polishing which does not modify the shape of the surface but eliminates any possible irregularity due to the ablation,
iii. Subtraction by computer of the elevation of the ablated surface from the surface prior to ablation, along the exact axis on which the ablation was applied, and with prior correction of errors of alignment and centring between the topographical measurements and the laser ablation and taking account of the errors of the topograph in the corneal periphery.

5. Method for obtaining a scale factor of the ablation depth obtained on the plastic material compared to the ablation depth expected in the corneal tissue, or other material, according to claim 1, section iii, for the surfaces **characterised in** claims 2 and/or 3, **characterised by** the measurement of the ablation depth at the apex of the surface difference of the surfaces after and before the ablation **characterised in** claims 2 and/or 3.

6. Method for obtaining the optimised profile for the treatment of the cornea, or forming by laser ablation of a pattern on a generic lens, according to claim 1, **characterised by** the product of any two-dimensional theoretical ablation profile with the inverse of the surface difference of the surfaces after and before the ablation **characterised in** claim 4 for the surface of claim 2 and by the scale factor **characterised in** claim 5.

7. Method for obtaining the optimised profile for the laser treatment of the cornea (or forming by laser ablation of a pattern on a generic lens) according to claim 1, **characterised by** the product of any two-dimensional theoretical ablation profile with the inverse of the surface difference of the surfaces after and before the ablation **characterised in** claim 4 for the surface of claim 3, by the theoretical ablation factor with which the surface **characterised in** 3 was treated, and by the scale factor **characterised in** claim 5.
